(19) Europäisches Patentamt
European Patent Office
Office européen des brevets

(11) Veröffentlichungsnummer: **0 015 385**
**B1**

(12) # EUROPÄISCHE PATENTSCHRIFT

(45) Veröffentlichungstag der Patentschrift: **12.05.82**

(51) Int. Cl.³: **C 07 C 121/407**, B 01 J 31/20

(21) Anmeldenummer: **80100511.7**

(22) Anmeldetag: **01.02.80**

(54) **Verfahren zur Herstellung von n-Propylcyanessigsäureestern und n-Äthylcyanessigsäureestern.**

(30) Priorität: **01.03.79 DE 2907873**

(43) Veröffentlichungstag der Anmeldung: **17.09.80 Patentblatt 80/19**

(45) Bekanntmachung des Hinweises auf die Patenterteilung: **12.05.82 Patentblatt 82/19**

(84) Benannte Vertragsstaaten: **BE CH DE FR GB IT NL**

(73) Patentinhaber: **DYNAMIT NOBEL AKTIENGESELLSCHAFT, Patentabteilung Postfach 1209, D-5210 Troisdorf, Bez. Köln (DE)**

(72) Erfinder: **Prange, Uwe, Dr., Porzer Strasse 2, D-5216 Niederkassel 3 (DE)**
Erfinder: **Schrage, Klaus, Dr., Pfarrer-Wichert-Strasse 53, D-5330 Königswinter 21 (DE)**
Erfinder: **Vogt, Wilhelm, Dr., Neuenhöfer Allee 54, D-5000 Köln-Sülz (DE)**

(56) Entgegenhaltungen:
**DE-A-2 403 483**
**DE-A-2 648 004**
**FR-A-1 313 360**
**FR-A-2 381 738**
**CHEMICAL ABSTRACTS, Band 74, Nr. 8, 1. März 1971, Seite 318, Nr. 41 946 j, Columbus, Ohio, USA**
**DERWENT JAPANESE PATENTS REPORT, Band 6, Nr. 36, 16. Oktober 1967, Derwent Publications, London, GB**
**BULLETIN OF THE CHEMICAL SOCIETY OF JAPAN, Band 40, Januar 1967, Seiten 135–144, Japan Publications Trading Co., Ltd., Tokyo, JP. A. MATSUDA: »The cobalt carbonyl-catalyzed hydroesterification of acrylonitrile with carbon monoxide and methanol in the presence of a small amount of hydrogen and alimited amount of pyridine«**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 015 385 B1

BUNDESDRUCKEREI BERLIN

# Verfahren zur Herstellung von n-Propylcyanessigsäureestern und n-Äthylcyanessigsäureestern

Die Erfindung betrifft die Herstellung von α-n-Propylcyanessigsäureestern aus 2-, 3- oder 4-Pentennitril sowie die Herstellung von α-n-Äthylcyanessigsäureester aus 3-Butennitril durch Umsetzung mit einem Alkohol und Kohlenmonoxid in Gegenwart von $Co_2(CO)_8$ und einem α-substituierten Pyridin als basisch wirkende Verbindung.

Cope et al. referieren in »Org. Reactions« IX (1957), 265, die Herstellung von n-Propylcyanessigsäureäthylester durch Umsetzung von Cyanessigsäureäthylester mit n-Propylbromid oder -jodid in Gegenwart von Na-Äthylat. Dieses bekannte Alkylierungsverfahren liefert jedoch in beträchtlichem Ausmaß Nebenprodukte und Abfallstoffe, wodurch das Verfahren unwirtschaftlich wird.

Die Hydrocarbalkoxylierung von 3- und 4-Pentennitril in Gegenwart von Metallcarbonylen und einer einen 5- oder 6-gliedrigen, stickstoffhaltigen Ring enthaltenden heterocyclischen Verbindung wird in der DE-OS 2 541 640 beschrieben. Reaktionsprodukte sind jedoch 5-Cyanvaleriansäureester. Die Bildung von Propylcyanessigsäureester wird nicht erwähnt.

Weiterhin ist es bekannt, Acrylnitril mit Äthanol oder Propanol oder Isopropanol und CO in Gegenwart von $Co_2(CO)_8$ umzusetzen (JA-AS 31651/70). Neben anderen Reaktionsprodukten entsteht bis zu einer Ausbeute von höchstens 13% 2-Cyanpropionsäureäthyl- bzw. n-propyl- bzw. -isopropylester. Die Herstellung des Methylesters gelingt nach diesem Verfahren nicht.

Ferner ist ein Verfahren bekannt (JA-AS 17 246/67), bei dem Acrylnitril mit CO und Methanol in Gegenwart von $Co_2(CO)_8$, Pyridin und bis 10 Gew.-% $H_2$, bezogen auf Kohlenmonoxid, zu Gemischen von 2- und 3-Cyanpropionsäuremethylester, β-Cyanpropionaldehyddimethylacetal und Propionitril umgesetzt wird. Die gleichen Ergebnisse werden auch in Bull. Chem. Soc., Japan 40, 135–144 (1967) mitgeteilt.

Dieses zuletzt genannte Carbonylierungsverfahren liefert neben 2-Cyanpropionsäuremethylester beträchtliche Anteile an Nebenprodukten, deren Abtrennung auf Grund geringer Siedepunktdifferenzen Schwierigkeiten bereitet. Ein weiterer Nachteil liegt in der Notwendigkeit, Wasserstoff bis zu 10 Gew.-% dem Kohlenmonoxid zuzugeben.

Gegenstand der deutschen Patentanmeldung P 2 639 327 ist ein Verfahren zur Herstellung α-substituierter Cyanessigsäurealkylester durch Umsetzung eines α,β-olefinisch ungesättigten Nitrils mit einem Alkanol und Kohlenmonoxid in Gegenwart von $Co_2(CO)_8$ und einer basisch wirkenden organischen Verbindung bei erhöhten Temperaturen und erhöhtem Druck, daß als α,β-ungesättigte Nitrile Verbindungen der allgemeinen Formel

$$R^3{=}\overset{\overset{\displaystyle R^2}{|}}{C}{-}CN \qquad \mathrm{I}$$

in welcher $R^2$ ein Wasserstoffatom oder ein Methylrest ist, und in welcher $R^3$ einen Rest aus der Gruppe $CH_2$ oder $CHCH_3$ oder CHCN oder $CHC_6H_5$ oder $C(CH_3)_2$ bedeutet, daß als Alkohole solche der allgemeinen Formel

$$ROH \qquad \mathrm{II}$$

in welcher R ein primärer, sekundärer oder tertiärer gesättigter Alkylrest mit 1 bis 20 C-Atomen, vorzugsweise 1 bis 4 C-Atomen ist, eingesetzt werden und daß in Abwesenheit von Wasserstoff und in Gegenwart eines α-substituierten Pyridins gearbeitet wird.

Es wurde gefunden, daß aus 3-Butennitril die n-Äthylcyanessigsäureester und aus 2-, 3- oder 4-Pentennitril die n-Propylcyanessigsäureester herstellbar sind.

Das Verfahren wird am besten mit Kohlenmonoxid durchgeführt, das frei von Wasserstoff ist. Wichtig ist ferner, daß α-substituierte Pyridine als basische Verbindung eingesetzt werden. Durch die Anwendung von 2-, 3- oder 4-Pentennitril werden im Hinblick auf den Stand der Technik nach dem erfindungsgemäßen Verfahren auf einfache und wirtschaftliche Weise n-Propylcyanessigsäurealkylester in großer Ausbeute und Reinheit zugänglich. Nach dem Stand der Technik wäre hingegen anstelle der n-Propylcyanessigsäureester die Bildung von 2-Äthyl-3-cyanpropionsäureester sowie weitere Carbonsäureester wie Cyanvaleriansäureester zu erwarten gewesen.

Gegenstand der Erfindung ist daher das Verfahren zur Herstellung von α-n-Propylcyanessigsäureestern bzw. α-n-Äthylcyanessigsäureestern, welches dadurch gekennzeichnet ist, daß 2-Pentennitril, 3-Pentennitril, 4-Pentennitril, deren Mischungen bzw. 3-Butennitril in Gegenwart von $Co_2(CO)_8$ und einem α-substituierten Pyridin als basisch wirkender Verbindung mit einem Alkohol und Kohlenmonoxid umgesetzt werden.

Aus 3- und 4-Pentennitril bzw. 3-Butennitril entsteht unerwartet ganz überwiegend ebenfalls die n-Propylcyanessigsäureester bzw. n-Äthylcyanessigsäureester neben nur wenig ω-Cyancarbonsäureester, d. h. es tritt offenbar eine Isomerisierung ein, so daß hier ein neues Reaktionsprinzip vorliegt.

Das zur Herstellung von n-Propylcyanessigsäureestern verwendete 2-, 3- oder 4-Pentennitril entsteht u. a. bei der Addition von Blausäure an Butadien in Gegenwart von Nickelkomplexverbindungen.

Das 2-, 3- oder 4-Pentennitril oder deren Gemische können rein oder als ein Fraktionsschritt eingesetzt werden, der noch andere Stoffe, wie sie bei der Herstellung von 2-, 3- oder 4-Pentennitril anfallen, enthält. Als Begleitstoffe kommen besonders 2-Methyl-2-butennitril und/oder 2-Methyl-3-butennitril in Frage. In diesen Ausgangsstoffen sollen die Gehalte von 2-Pentennitril und/oder 3-Pentennitril mindestens 50 Gew.-%, bevorzugt mehr als 75 Gew.-% betragen.

Als Alkohole können primäre, sekundäre oder tertiäre gesättigte Alkohole mit 1—20 C-Atomen, vorzugsweise 1 bis 4 C-Atomen, eingesetzt werden. Genannt seien z. B. Methanol, Äthanol, n oder Iso-Propanol, Butanole, Hexanole oder verzweigte Alkohole wie 2-Äthylhexanol und dgl. Im allgemeinen wird der Alkohol im Überschuß, beispielsweise im 2- bis 6fachen molaren Überschuß, bezogen auf eingesetztes ungesättigtes Nitril, verwendet.

Der Kohlenmonoxid kann im Druckbereich von 50 bis 250 bar angewendet werden. Höhere Drücke sind möglich, aber nicht notwendig. Vorzugsweise wird im Druckbereich von 100 bis 180 bar Kaltdruck gearbeitet. Das Kohlenmonoxid kann Inertgas, wie z. B. $N_2$, enthalten.

Als Katalysator wird $Co_2(CO)_8$ verwendet. Er wird, bezogen auf ungesättigtes Nitril, im Molverhältnis von 1 : 10 bis 1 : 1000, vorzugsweise 1 : 50 bis 1 : 500 eingesetzt.

Es ist zweckmäßig, $\alpha$-substituierte Pyridine einzusetzen, deren Siedepunkte in einem Bereich liegen, daß eine leichte destillative Trennung von dem Endprodukt möglich ist.

Die im Sinne der Erfindung geeigneten $\alpha$-substituierten Pyridine sind solche der allgemeinen Formel

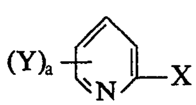

Der Substituent X kann ein primärer oder sekundärer oder tertiärer $C_1$- bis $C_4$-Alkylrest, der ggf. olefinisch ungesättigt ist, oder ein ggf. alkylsubstituierter Benzylrest, oder Halogen, oder ein Phenylrest, oder ein $\alpha$-Pyridylrest sein.

Y kann ein $C_1$- bis $C_4$-Alkylrest (primär, sekundär oder tertiär, gesättigt) oder Halogen sein.

a ist 0 bis 2. Bei a = 2 können die Substituenten Y gleich oder ungleich sein.

Bevorzugt sind die Substituenten Y (bzw. der Substituent Y) in 4- und/oder 6-Stellung an dem Kern angeordnet. Y kann gleich oder ungleich X sein.

Genannt seien z. B. $\alpha$-Picolin, 2,4-Lutidin, 2,6-Lutidin, 2,4,6-Kollidin, 2-Äthylpyridin, 2-n-Propyl- bzw. 2-Isopropylpyridin, 2-n-Butyl- bzw. 2-tert.-Butylpyridin, 2-Vinylpyridin, 2-Propenylpyridin, 2-Chlorpyridin, 2-Brom- bzw. 2-Jodpyridin, 2-Benzylpyridin, 2-Phenylpyridin, $\alpha,\alpha'$-Bipyridyl u. dgl. $\alpha$-Picolin, 2,4- und 2,6-Lutidin, 2,4,6-Kollidin, 2-Äthylpyridin, 2-Vinylpyridin und 2-Chlorpyridin haben sich als gut selektiv wirkende Stoffe erwiesen.

$\alpha$-alkylsubstituierte Pyridine, insbesondere methyl- oder äthylsubstituierte, werden bevorzugt, insbesondere $\alpha$-Picolin und 2,4-Lutidin.

Die $\alpha$-alkylsubstituierten Pyridine werden, bezogen auf ungesättigtes Nitril, im molaren Verhältnis (Pyridinabkömmlinge zu ungesättigtem Nitril) von 1 : 1 bis 1 : 100, vorzugsweise 1 : 10 bis 1 : 50 eingesetzt.

Die Reaktionstemperatur kann zwischen 100 und 250° C, vorzugsweise zwischen 120 und 220° C liegen.

Im allgemeinen wird die Reaktion so durchgeführt, daß die Komponenten in einem Autoklav vorgelegt werden und unter Rühren und Druck auf die gewünschte Reaktionstemperatur erhöht wird.

Es ist jedoch auch möglich, das ungesättigte Nitril bei erhöhtem Druck und erhöhter Temperatur den übrigen Reaktionsteilnehmern zuzudosieren.

Das Reaktionsprodukt n-Propyl-cyanessigsäurealkylester läßt sich leicht aus dem Reaktionsgemisch abtrennen. Ein übliches Verfahren ist z. B. die fraktionierte Destillation, wodurch auch der überschüssige Alkohol sowie das $\alpha$-substituierte Pyridin zurückgewonnen werden können.

Die durch das erfindungsgemäße Verfahren zugänglichen n-Propylcyanessigsäurealkylester sind wertvolle chemische Zwischenprodukte. So kann z. B. durch Alkylierung mit n-Propylhalogenid der Di-n-propylcyanessigsäureester hergestellt werden, der nach der DE-OS 2 721 264 zu Dipropylessigsäure umgesetzt werden kann. Andererseits kann die Nitrilgruppe verseift werden und man erhält Propylmalonsäuredialkylester.

## Beispiel 1

In einem mit einem Hubrührer versehenen 1-l-Edelstahlautoklav werden bei Raumtemperatur 81 g (1 Mol) 2-Pentennitril, 10 g $Co_2(CO)_8$, 20 g $\alpha$-Picolin und 400 ml Äthanol unter Stickstoffatmosphäre vorgelegt. Danach wird durch Spülen mit Kohlenmonoxid der Stickstoff entfernt und ein CO-Kaltdruck von 200 bar eingestellt. Anschließend wird unter Rühren auf 150° C erhitzt und diese Temperatur 3 Stunden gehalten. Nach dem Erkalten des Reaktionsgutes wird der verbleibende CO-Druck abgelassen und der Ansatz durch Destillation aufgearbeitet. Der Umsatz an 2-Pentennitril liegt bei 90% und es werden 125,3 g n-Propylcyanessigsäureäthylester gewonnen (Ausbeute 90%).

## Beispiel 2

Analog Beispiel 1, jedoch mit nur 5 g $Co_2(CO)_8$ werden die Reaktionskomponenten 2 Stunden bei 160° C gehalten. Die Aufarbeitung ergibt bei einem Umsatz von 87% 117,2 g n-Propylcyanessigsäureäthylester (Ausbeute 87%).

Beispiel 3

Analog Beispiel 1, jedoch mit 5 g $Co_2(CO)_8$, 15 g $\alpha$-Picolin und 100 g einer Fraktion, die 85% 2-Pentennitril neben 2-Methyl-2-butennitril und 2-Methyl-3-butennitril enthielt, konnte nach 6 Stunden Reaktion bei 200 bar CO-Kaltdruck und 150°C 119 g n-Propylcyanessigsäureäthylester isoliert werden. Bei 80% Umsatz lag die Ausbeute bei 90%. 2-Methyl-2-butennitril und 3-Methyl-3-butennitril wurden unverändert zurückgewonnen.

Beispiel 4

Analog Beispiel 3, jedoch mit nur 140 bar CO-Kaltdruck konnten bei einem Umsatz von 72% 101 g n-Propylcyanessigsäureäthylester erhalten werden (Ausbeute 86%).

Beispiel 5

Analog Beispiel 4, jedoch mit iso-Propanol anstelle von Äthanol, wurde nach der destillativen Aufarbeitung 106 g n-Propylcyanessigsäureisopropylester erhalten. Bei einem Umsatz von 74% lag die Ausbeute somit bei 81,5%.

n-Propylcyanessigsäureisopropylester
$Kp_{14}$: 107°C
$C_9H_{15}NO_2$,
Mol-Gew.: 169,225; Mol-Gew.: 169 MS
C, H, N berechnet
C=63,88%, H=8,94%, N=8,28%
C, H, N gefunden
C=63,89%, H=9,34%, N=8,18%

Beispiel 6

Analog Beispiel 4, jedoch mit n-Butanol anstelle von Äthanol, wurden 128,5 g n-Propylcyanessigsäure-n-butylester isoliert. Bei einem Umsatz von 75% lag die Ausbeute bei 89%.

n-Propylcyanessigsäure-n-butylester
$Kp_{14}$: 126–7°C
$C_{10}H_{17}NO_2$
Mol-Gew.: 183,25; Mol-Gew.: 183 MS
C, H, N berechnet
C=65,54%, H=9,40%, N=7,69%
C, H, N gefunden
C=65,37%, H=9,57%, N=7,67%

Beispiel 7

Analog Beispiel 1, jedoch mit 81 g (1 Mol) 3-Pentennitril anstelle von 2-Pentennitril und einer Reaktionstemperatur von 160°C und einer Reaktionszeit von 5 Stunden, werden bei einem Umsatz von 63% 81% n-Propylcyanessigsäureäthylester und 7,5% 5-Cyanvaleriansäureäthylester isoliert. 4-Pentennitril kann in entsprechender Weise umgesetzt werden.

Beispiel 8

Entsprechend Beispiel 1 wird 1 Mol 3-Butennitril (= Allylcyanid) bei einer Reaktionstemperatur von 140°C umgesetzt. Die Ausbeute besteht zu 95% aus n-Äthylcyanessigsäureäthylester und zu nur 1% aus 4-Cyanbuttersäureäthylester.

**Patentansprüche**

1. Verfahren zur Herstellung von $\alpha$-n-Propylcyanessigsäureestern bzw. $\alpha$-n-Äthylcyanessigsäureestern, welches dadurch gekennzeichnet ist, daß 2-Pentennitril, 3-Pentennitril, 4-Pentennitril, deren Mischungen bzw. 3-Butennitril in Gegenwart von $Co_2(CO)_8$ und einem $\alpha$-substituierten Pyridin als basisch wirkender Verbindung mit einem Alkohol und Kohlenmonoxid umgesetzt werden.

2. Verfahren nach Anspruch 1, dadurch gekennzeichnet, daß als 2- oder 3-Pentennitril Additionsprodukte von Blausäuren an Butadien eingesetzt werden, deren Gehalt an 2-Pentennitril oder 3-Pentennitril oder 2- und 3-Pentennitril zusammen mindestens 50 Gew.-%, bevorzugt mehr als 75%, betragen.

**Claims**

1. Process for the preparation of alpha-n-propylcyano acetic acid esters or alpha-n-ethylcyano acetic acid esters, which is characterized in that 2-pentenenitrile, 3-pentenenitrile, 4-pentenenitrile, their mixtures or 3-butenenitrile are reacted with an alcohol and carbon monoxide in the presence of $Co_2(CO)_8$ and an alpha-substituted pyridine as basically acting compound.

2. Process according to claim 1, characterised in that there are employed as 2- or 3-pentenenitrile, addition products of hydrocyanic acid to butadiene, whose content of 2-pentenenitrile or 3-pentenenitrile or 2- and 3-pentenenitrile together amounts to at least 50 weight percent, preferably more than 75 percent.

**Revendications**

1. Procédé pour la préparation d'esters de l'acide alpha-n-propylcyanoacetique ou d'esters de l'acide alpha-n-éthylcyanoacétique, caracterisé en ce qu'on fait réagir du 2-pentenenitrile, du 3-pentenenitrile, du 4-pentenenitrile, leurs mélanges ou du 3-butenenitrile, en présence de $Co_2(CO)_8$ et d'une pyridine alpha-substituée comme composé a acitivité, basique, avec un alcool et du monoxyde de carbone.

2. Procédé selon la revendication 1, caracterisé en ce que on utilise comme 2- ou 3-pentenenitrile des producits d'additions d'acides cyanhydriques sur du butadiene, dont le teneur en 2-pentenenitrile ou en 3-pentenenitrile, ou a la fois en 2- et 3-pentenenitrile, représente au moins 50 pourcent en poids, et de préférence plus de 75 pourcent.